# EUROPEAN PATENT APPLICATION

(11) **EP 4 531 051 A1**
(43) Date of publication of application: **02.04.2025**
(21) Application number: 24203360.3
(22) Date of filing: 27.09.2024
(51) Int. Cl.: G16H 20/17, G16H 40/63, A61M 5/172

(54) **INFUSION PUMP WITH MULTI-CGM COMPATIBILITY**

(30) Priority: 29.09.2023 US 202318478552
(71) Applicant: Tandem Diabetes Care, Inc., San Diego, CA 92130 (US)
(72) Inventor: KRUSE, Geoff, San Diego, 92130 (US); JUSTER, Josh, San Diego, 92130 (US); TRAN, Kuniaki, San Diego, 92130 (US); JUNG, Christopher, San Diego, 92130 (US); LEE, Dwight, San Diego, 92130 (US); HARRIS, Paul, San Diego, 92130 (US); YU, Kristal, San Diego, 92130 (US); CAHILL, Lori, San Diego, 92130 (US)
(74) Representative: HGF

(57) **Abstract**

Embodiments disclosed herein relate to systems and methods for adapting ambulatory infusion pump systems to operate with different types of continuous glucose monitoring (CGM) sensors. Infusion pump systems as disclosed herein can include software configured to store characteristics for different types of CGM sensors and can interface with a given type of CGM sensor based on specific characteristics and requirements of the given type of sensor.

## Description

### CROSS-REFERENCE TO RELATED APPLICATION

This application claims the benefit of and priority to U.S. Patent Application No. 18/478,552, filed September 29, 2023, the disclosure of which is hereby incorporated by reference in its entirety.

### TECHNICAL FIELD

The present disclosure is directed generally to systems and methods relating to portable infusion pumps and more particularly for portable infusion pumps that interface with continuous glucose monitoring (CGM) sensors.

### BACKGROUND

There are a wide variety of medical treatments that include the administration of a therapeutic fluid in precise, known amounts at predetermined intervals. Devices and methods exist that are directed to the delivery of such fluids, which may be liquids or gases, are known in the art.

One category of such fluid delivery devices includes insulin injecting pumps developed for administering insulin to patients afflicted with type 1, or in some cases, type 2 diabetes. Some insulin injecting pumps are configured as portable or ambulatory infusion devices can provide continuous subcutaneous insulin injection and/or infusion therapy as an alternative to multiple daily injections of insulin via a syringe or an insulin pen. Such pumps are worn by the user and may use replaceable cartridges. In some embodiments, these pumps may also deliver medicaments other than, or in addition to, insulin, such as glucagon, pramlintide, and the like. Examples of such pumps and various features associated therewith include those disclosed in U.S. Patent Publication Nos. 2013/0324928 and 2013/0053816 and U.S. Patent Nos. 8,287,495; 8,573,027; 8,986,253; and 9,381,297, each of which is incorporated herein by reference in its entirety.

Ambulatory infusion pumps have generally been controlled by a user interface provided on the pump. With the proliferation of handheld electronic devices, such as mobile phones (e.g., smartphones), there is a desire to be able to remotely utilize such devices, as well as dedicated wireless controllers designed to work with one or more infusion pumps and/or types of infusion pumps, to optimize usage of infusion pumps. These remote controllers would enable a pump to be monitored, programmed and/or operated more privately, more conveniently and more comfortably. Accordingly, one potential use of dedicated remote devices and handheld consumer electronic devices (such as smartphones, tablets and the like) is to utilize such devices as controllers for remotely programming and/or operating infusion pumps.

While different portable infusion pumps can be configured to connect to a plurality of different types of CGM sensors, it remains a challenge for a single portable infusion pump software to connect to multiple types of CGM sensors. This is because each CGM vendor and version has its own communication protocols, parameters, security measures, Bluetooth^{®} Connectivity models, sensor operating intervals, cybersecurity models, and overall characteristics. As of yet, it remains out of reach to normalize the various differences between each CGM type such that a single unified software application can direct and connect to a plurality of types of CGM sensors.

### SUMMARY

Embodiments disclosed herein relate to systems and methods for adapting ambulatory infusion pump systems to operate with different types of continuous glucose monitoring (CGM) sensors. Infusion pump systems as disclosed herein can include software configured to store characteristics for different types of CGM sensors and can interface with a given type of CGM sensor based on specific characteristics and requirements of the given type of sensor.

In embodiments, an ambulatory infusion pump system can include a pump mechanism configured to deliver insulin to a user, a communications interface configured to receive data indicative of glucose levels of a user from one or more continuous glucose monitoring (CGM) sensors and a memory configured to store characteristics for each of a plurality of types of CGM sensors. At least one processor can be configured to receive an indication that a user will be using a type of sensor from the plurality of types of sensors. The at least one processor can the access the stored characteristics in memory for the type of sensor and configure the system to interface with the type of sensor based on the stored characteristics.

In embodiments, an ambulatory infusion pump system can include a pump mechanism configured to deliver insulin to a user, a communications interface configured to receive data indicative of glucose levels of a user from one or more continuous glucose monitoring (CGM) sensors and a memory configured to store characteristics for each of a plurality of types of CGM sensors. A user interface can be configured to display a menu screen enabling a user to select a type of CGM sensor from a list of the plurality of types of CGM sensors and at least one processor can be configured to interface with the type of sensor based on the stored characteristics.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
FIG. 1 depicts an embodiment of a pump system according to the disclosure.
FIG. 2 depicts a block diagram representing an embodiment of a pump system according to the disclosure.
FIGS. 3A-3B depicts an embodiment of a pump system according to the disclosure.
FIG. 4 depicts an embodiment of a pump system according to the disclosure.
FIGS. 5A-5B depict remote control devices for a pump system according to embodiments of the disclosure.
FIG. 6 depicts a schematic representation of a pump system according to an embodiment of the disclosure.
FIGS. 7A-7H depict graphical user interface screens according to an embodiment of the disclosure.
FIG. 8 depicts a flowchart of method for connecting to a CGM sensor according to embodiments of the disclosure.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover all modifications, equivalents, and alternatives falling within the spirit and scope of the subject matter as defined by the claims.

### DETAILED DESCRIPTION OF THE DRAWINGS

The following detailed description should be read with reference to the drawings in which similar elements in different drawings are numbered the same. The drawings, which are not necessarily to scale, depict illustrative embodiments and are not intended to limit the scope of the invention.

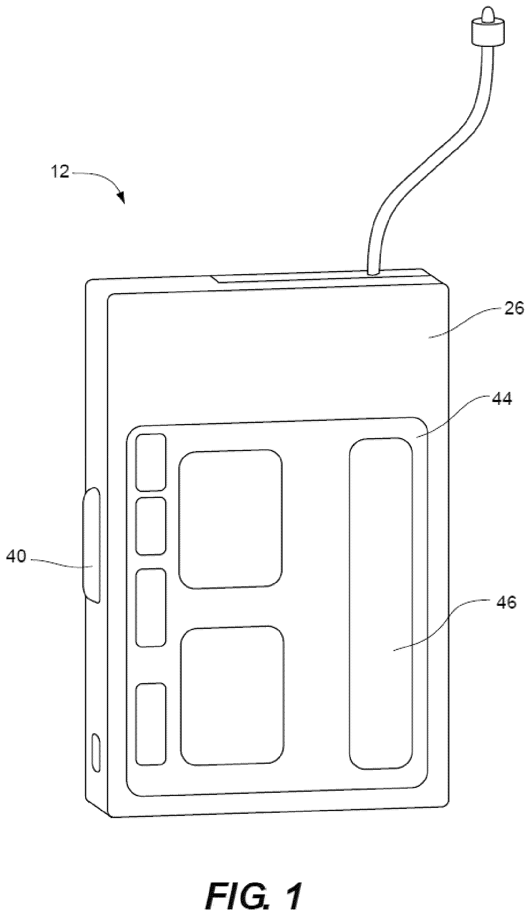

FIG. 1 depicts an exemplary medical device that can be used with embodiments of the disclosure. In this embodiment, the medical device is configured as a pump 12, such as an infusion pump, that can include a pumping or delivery mechanism and reservoir for delivering medicament to a patient and an output/display 44. The type of output/display 44 may vary as may be useful for a particular application. The output/display 44 may include an interactive and/or touch sensitive screen 46 having an input device such as, for example, a touch screen comprising a capacitive screen or a resistive screen. The pump 12 may additionally include a keyboard, microphone, or other input device known in the art for data entry, which may be separate from the display. The pump 12 may also include a capability to operatively couple to one or more blood glucose meters (BGMs) or continuous blood glucose monitors (CGMs) and/or one or more secondary devices such as a remote display, a remote control device, a laptop computer, personal computer, tablet computer, a mobile communication device such as a smartphone, a wearable electronic watch, smart ring, electronic health or fitness monitor, or personal digital assistant (PDA), a CGM display etc.

In one embodiment, the medical device can be a portable pump configured to deliver insulin to a patient. Further details regarding such pump devices can be found in U.S. Patent No. 8,287,495, which is incorporated herein by reference in its entirety. In other embodiments, the medical device can be an infusion pump configured to deliver one or more additional or other medicaments to a patient.

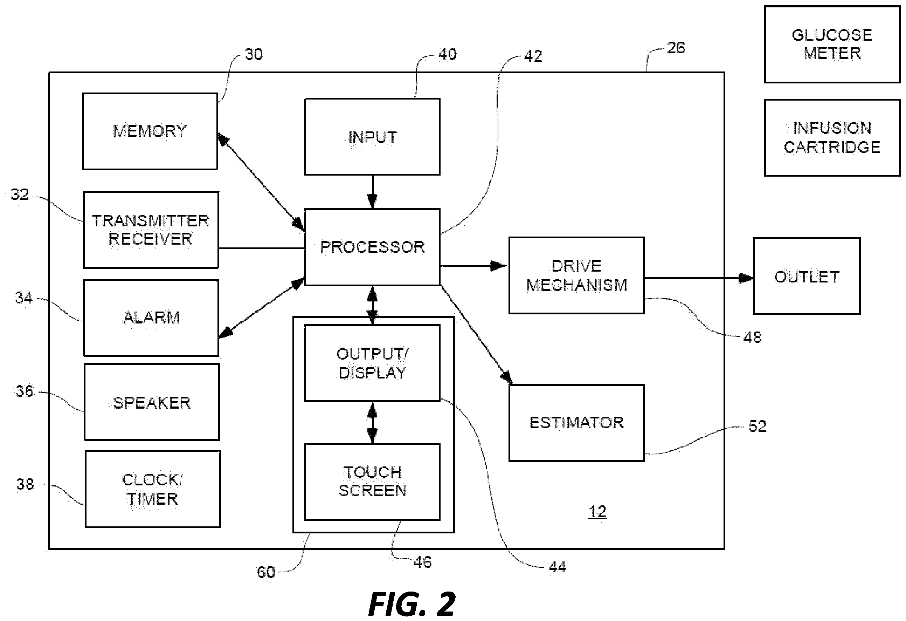

FIG. 2 illustrates a block diagram of some of the features that can be used with embodiments, including features that may be incorporated within the housing 26 of a medical device such as a pump 12. The pump 12 can include a processor 42 that controls the overall functions of the device. The infusion pump 12 may also include, e.g., a memory device 30, a transmitter/receiver 32, an alarm 34, a speaker 36, a clock/timer 38, an input device 40, a user interface suitable for accepting input and commands from a user such as a caregiver or patient, a drive mechanism 48, an estimator device 52 and a microphone (not pictured). One embodiment of a user interface as shown in FIG. 2 is a graphical user interface (GUI) 60 having a touch sensitive screen 46 with input capability. In some embodiments, the processor 42 may communicate with one or more other processors within the pump 12 and/or one or more processors of other devices, for example, a continuous glucose monitor (CGM), display device, smartphone, etc. through the transmitter/receiver. The processor 42 may also include programming that may allow the processor to receive signals and/or other data from one or more input devices, such as sensors that may sense pressure, temperature and/or other parameters.

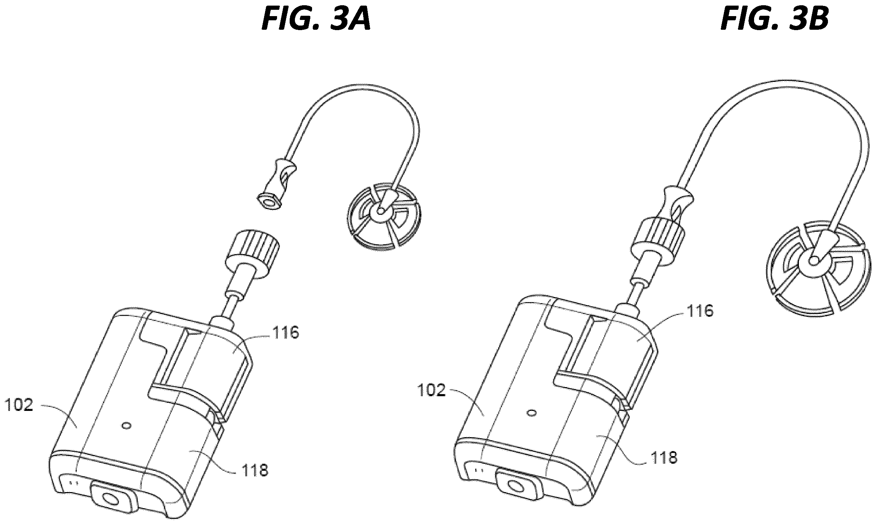

Figures 3A-3B depict a second infusion pump that can be used in conjunction with one or more embodiments of the ambulatory infusion pump system of the present disclosure. Pump 102 includes a pump drive unit 118 and a medicament cartridge 116. Pump 102 includes a processor that may communicate with one or more processors within the pump 102 and/or one or more processors of other devices such as a remote device (e.g., a CGM device), a remote control device, or a consumer electronic device (e.g., laptop computer, personal computer, tablet computer, smartphone, electronic watch, smart ring, electronic health or fitness monitor, or personal digital assistant). The processor 42 may also include programming to receive signals and/or other data from an input device, such as, by way of example, a pressure sensor, a temperature sensor, or the like. Pump 102 also includes a processor that controls some or all of the operations of the pump. In some embodiments, pump 102 receives commands from a separate device for control of some or all of the operations of the pump. Such separate device can include, for example, a dedicated remote control device or a consumer electronic device such as a smartphone having a processor executing an application configured to enable the device to transmit operating commands to the processor of pump 102. In some embodiments, processor can also transmit information to one or more separate devices, such as information pertaining to device parameters, alarms, reminders, pump status, etc. Such separate device can include any remote display, remote control device, or a consumer electronic device as described above. Pump 102 can also incorporate any or all of the features described with respect to pump 12 in Figure 2. In some embodiments, the communication is effectuated wirelessly, by way of example only, via a near field communication (NFC) radio frequency (RF) transmitter or a transmitter operating according to a "Wi-Fi" or Bluetooth^{®} protocol, Bluetooth^{®} low energy protocol or the like. Further details regarding such pumps can be found in U.S. Patent No. 10,279,106 and U.S. Patent Publication Nos. 2016/0339172 and 2017/0049957, each of which is hereby incorporated herein by reference in its entirety.

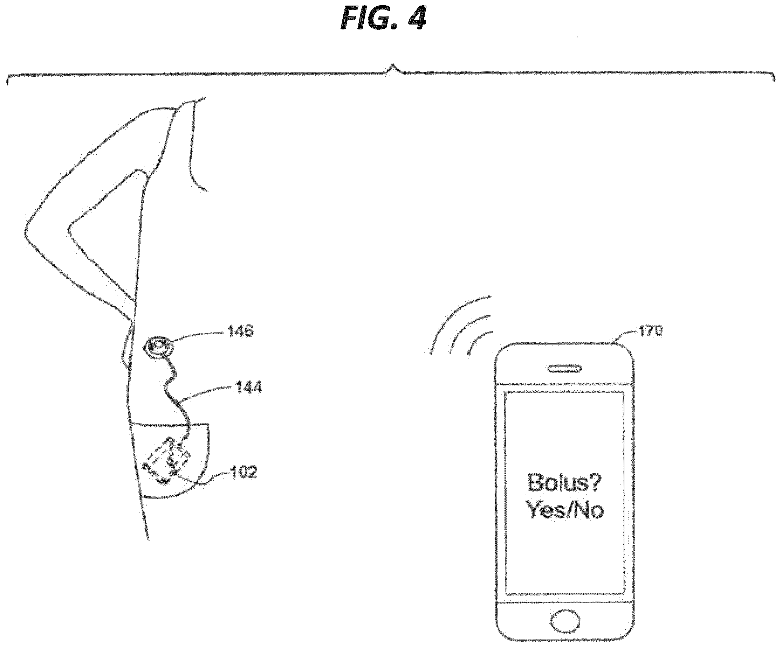

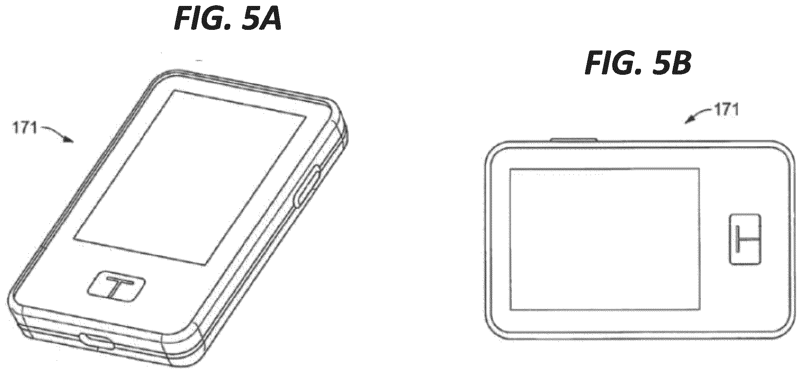

Referring to Figs. 4-5B, one or more remote control devices 170, 171 can be used to communicate with the processor of pump 12 and/or pump 102 to control delivery of medicament and transfer data with pump via a wired or a wireless electromagnetic signal, such as via, e.g., a near field communication (NFC) radio frequency (RF) modality or other RF modalities such as Bluetooth^{®}, Bluetooth^{®} low energy, mobile or Wi-Fi communication protocols, for example, according to embodiments of the present disclosure. Such a remote control can include, for example, a mobile communication device 170, such as a smart phone (as depicted in Fig. 4) executing a software application for control of the pump, a dedicated remote controller 171 (as depicted in Figs. 5A-5B), a wearable electronic watch, smart ring, electronic health or fitness monitor or personal digital assistant (PDA), etc., or a tablet, laptop or personal computer. Such communications between (and among) the one or more remote control devices 170, 171 and pump 102 may be one-way or two-way for, e.g., effective transfer of data among the devices and the pump, control of pump operations, updating software on the devices and/or pump, and allowing pump-related data to be viewed on the devices and/or pump.

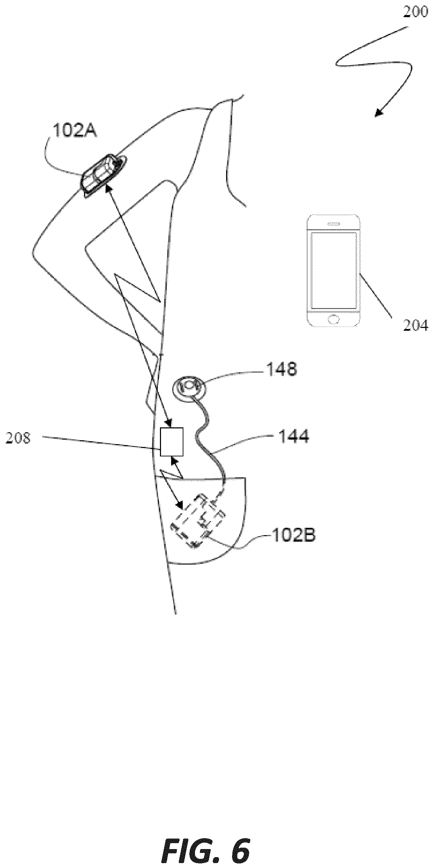

FIG. 6 depicts a schematic representation of a pump system 200 according to an embodiment. System 200 includes a user-wearable infusion pump such as pump 12 or pump 102 described above. In embodiments, a user can alternatively wear the pump 102A directly on the body or place the pump 102B in the user's pocket or other location near the body with infusion tubing 144 extending to an infusion set 148 on the user's body. The system 200 also includes a continuous glucose monitoring (CGM) sensor with a corresponding transmitter 208. The CGM sensor obtains measurements relating to glucose levels in the body and the transmitter communicates that information to the pump 102A/B. The pump 208 can then use the glucose data in making therapy determinations. The system can also include a one or more devices such as a smartphone 204 or other multi-purpose consumer electronic device capable of operating a software application to communicate with and/or control the pump and, alternatively or additionally, a dedicated remote control device designed specifically for use with pump 102A/102B. The smartphone 204 or other remote control device can in some embodiments also be capable of communication with CGM sensor/transmitter 208. In addition, the pump 102A/B and/or smartphone 204 or other remote control device can optionally communicate with additional devices such as, for example, a blood glucose meter or other analyte sensing device, an activity or other health monitor, etc.

Although depicted with the multi-purpose consumer electronic device 204 being a smartphone, in various embodiments the consumer electronic device can alternatively or additional include one or more of a wearable electronic watch, such as a smartwatch, a smart ring, electronic health or fitness monitor, personal digital assistant (PDA), or a tablet, laptop or personal computer, etc. A multi-purpose consumer electronic device can be any device sold to consumers and used for a variety of functions and which can be configured or programmed to communicate with and/or control an infusion pump as one of said functions. In some embodiments, systems as described herein may include more than one multi-purpose consumer electronic device configured for communication with the infusion pump (e.g., a smartphone and a smart watch).

As noted above, while different portable infusion pumps can be configured to connect to a plurality of different types of CGM sensors, it remains a challenge for a single portable infusion pump software to connect to multiple types of CGM sensors because each CGM vendor and version has its own communication protocols, parameters, security measures, Bluetooth^{®} Connectivity models, sensor operating intervals, cybersecurity models, and overall characteristics. Embodiments disclosed herein provide portable infusion pumps and/or remote control devices that are programmed to normalize the various differences between each CGM type such that the pump can interchangeably connect to a plurality of types of CGM sensors. A user interface or series of menu screens can also be provided to guide a user through setting up a pump to operate with a plurality of different types of CGM sensors.

In an example, a software application executing on an infusion pump and/or a remote control device is programmed to execute instructions which generate and store varying protocols and parameters relating to each type of CGM sensor to which a pump may be connected. As an example, stored instructions may relate to varying characteristics, including at least communication protocols, parameters, sensor operating interval (e.g., 1 minute interval versus 5 minute interval, etc.), security measures, cybersecurity models, and Bluetooth connectivity models. As different types of CGM sensors operate under differing characteristics, pump software can be configured to generate and store differing operability conditions and characteristics based on selected type CGM and execute instructions to based on differing operability conditions and characteristics.

As will be discussed in more detail below, the pump and/or remote control device may be configured to display a menu providing the user options to select a type of sensor from multiple CGM sensor types, to switch operation and/or communicate and transfer stored data between CGM sensors, to cease operation of an individual CGM sensor, etc. CGM sensors may be selected by session, where a previous CGM session has been forcibly ended or expired, and where a subsequent CGM session is selected. The subsequent CGM session may inherit data detected in the previous CGM session, either by means of transmitting data from the previous CGM session to the subsequent CGM session or by connecting the subsequent CGM session to stored memory, such as the cloud. Down stream display and use of data can be enabled, wherein further processing of detected data can occur by storing, transmitting, and processing CGM sensor statistics as detected across different CGM sensors over different CGM sessions.

One example of different operating characteristics for differing types of CGM sensors can include operating under different Bluetooth^{®} or other communication protocols (a radio frequency (RF) communication link, an Infrared communication link, or a wireless local area network (WLAN), Near-Field Communication (NFC), etc.) For example, certain types of CGM sensors can be configured to awaken in intervals, for example in increments of up to every 5 minutes to detect blood glucose level of the user, transmit the detected data to a corresponding connected pump (and/or remote control device) for display or for further processing. In such a type of CGM sensor, a new connection between the pump and/or remote control device and the CGM sensor is made at every (e.g., five minute) interval that the CGM sensor is awakened. Other types of sensors may maintain a constant Bluetooth connection, in which the sensor is connected at the start of a CGM session and the sensor pushes data to the pump and/or remote control at regular intervals (e.g., one minute). Other modes of communication are also possible. The software disclosed herein can be configured to know which type of protocol is employed by different types of sensors and to automatically communication with a selected type of sensor according to the known protocol for that sensor.

Sensor activation can also differ by type of CGM sensor. Some CGM sensors may be activated using standard Bluetooth communication, wherein sensor presence is advertised when Bluetooth communication on the CGM sensor is enabled and the sensor is detected in a pairing menu of the pump and/or the remote control. Different sensors may then use different authentication mechanisms, such as manual user entry of a transmitter ID, authentication code, or password, on the pump and/or remote control can occur to confirm Bluetooth pairing. Other sensor types may use Bluetooth pairing while further authenticating NFC authentication, such as by detecting a hardware identifier (e.g., serial number) via NFC. Software disclosed herein can be configured to know what type of activation protocol is required for each type of sensor and to guide the user through the appropriate activation type for a given sensor selected by the user.

The type of data that is transmitted from the CGM sensor to the pump and/or remote control device can also differ based on type of selected CGM sensor. For example, one type of CGM sensor may transmit measured glucose levels of the user (e.g., 300 md/dL) that can be directly utilized in therapy calculations. Another type of sensor may transmit an electrical signal that correlate to glucose levels, wherein the electrical signals are further processed by the pump and/or remote control device to calculate a glucose level indicated by the signal for use in therapy calculations. Pump and/or remote control software disclosed herein can be programmed to know which type of data to expect from a given type of sensor and to process such data accordingly.

CGM sensors can further differ in cybersecurity protocols based on type of CGM sensor. For example, each type of CGM sensor generally has its own way to mutually authenticate the CGM sensor to paired devices. Authentication may occur through manual entry of NFC generated transmitter ID, through varying public key infrastructure (PKI) protocols, and/or through any various means of secure authentication. At the core, each type of CGM sensor aims to authenticate, albeit differently, that it is transmitting trustworthy and secure data. As such, software must be cognizant of varying authentication measures and recognize and respond to them appropriately. As an example, software must be able to verify that data transmitted from any type of CGM sensor is from that CGM sensor based on protocols conventionally utilized by vendors affiliated with that CGM sensor. Further, software must be able to ascertain whether any other source is transmitting data to corrupt the data transmitted by the CGM sensor.

In addition, software must recognize that different types of CGM sensors may encrypt data in unique ways in order to protect the integrity of the transmitted data, and therefore software must be able to process a plurality of configurations of encryption methods.

Operational differences also exist between various types of sensors, such as sensor operating intervals, setting of alarms and alerts and corresponding alarm and alert trigger types, performance characteristics (such as acceptable noise levels, accuracy levels, time of initial "warm up" period before CGM sensor is fully awakened, signal integrity, which can all differ based on differing type of CGM sensor) and must also be tracked by software application in order to effectively be capable of interfacing with a plurality of types of CGM sensors.

For example, CGM sensor warm up periods following insertion into the body can range from, for example, 30 minutes to 2 hours, during which the CGM sensor does not transmit data relating to blood glucose levels. Some pumps systems may modify therapy protocols during these warmup periods and then automatically transition to therapy based on the glucose levels from the CGM sensor following the warmup period, so the software will need to be aware of the differing warmup periods for different sensor.

As noted above, different types of CGM sensors may provide data to the system at different intervals, such as, for example, every 5 minutes, every 1 minute, etc. The system software will need to reconcile these differences so that the system is functionally equivalent for the user. For example, a pump system will generally include certain alerts and alarms relating to CGM communications, data, etc. The system will ideally process data from different types of CGM sensors in the same way for determining whether an alert or alarm should be issued.

Additionally, different types of CGM sensors can differ in detection of range of blood glucose levels. For example, certain CGM sensors can range in detection of glucose levels between 40 milligrams per deciliter (mg/dL) to 400 mg/dL. Other types of CGM sensors can detect anywhere from 20 mg/dL to 600 mg/dL. Therefore, software application can calculate, based on the selected type of CGM sensor, whether constraints or filters over the selected data are required in order to calibrate detected data to coincide with triggers set in the overall system such that data can be processed similarly. For example, in order for the data to be processed in a common algorithm in the example given above, the second type of sensor (20 to 600 mg/dL) may be constrained to have minimum and maximum values of 40 to 400 mg/dL for use in system calculations.

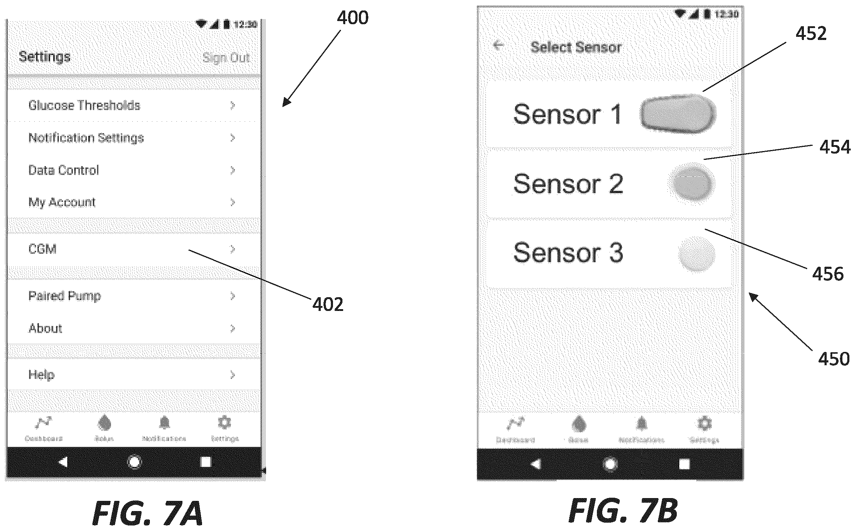

FIGS. 7A-7F depict menu screens that can be presented on a graphical user interface display of an infusion pump and/or a remote control device to guide the user in selecting and/or configuring for use a type of CGM sensor selected from multiple types of CGM sensors. For example, when a user does not have an active CGM session, a CGM menu item 402 can be accessible from a Settings menu screen 400 as shown in Figure 7A. Upon selection of the CGM menu item 402, a Select Sensor menu screen 450 can be displayed. The Select Sensor menu screen 450 can display menu items 452, 454, 456 for the various types of CGM sensors that are supported by the system. If a user is attempting to use a type of CGM sensor that is not supported by the system, it will not be displayed. As shown in Figure 7B, in some embodiments each sensor can be identified with one or more of text reciting the name of the type of sensor and a graphical image depicting the sensor (which may aid more inexperienced users).

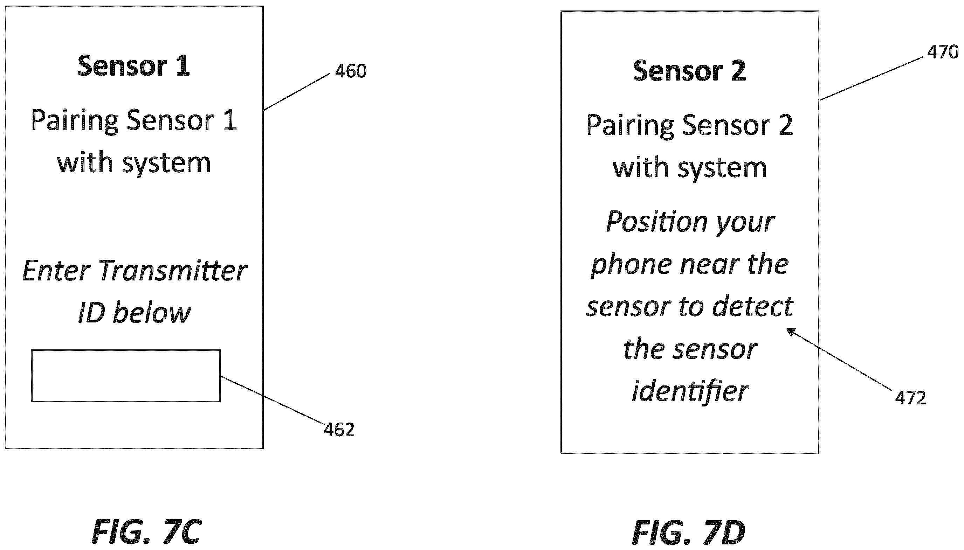

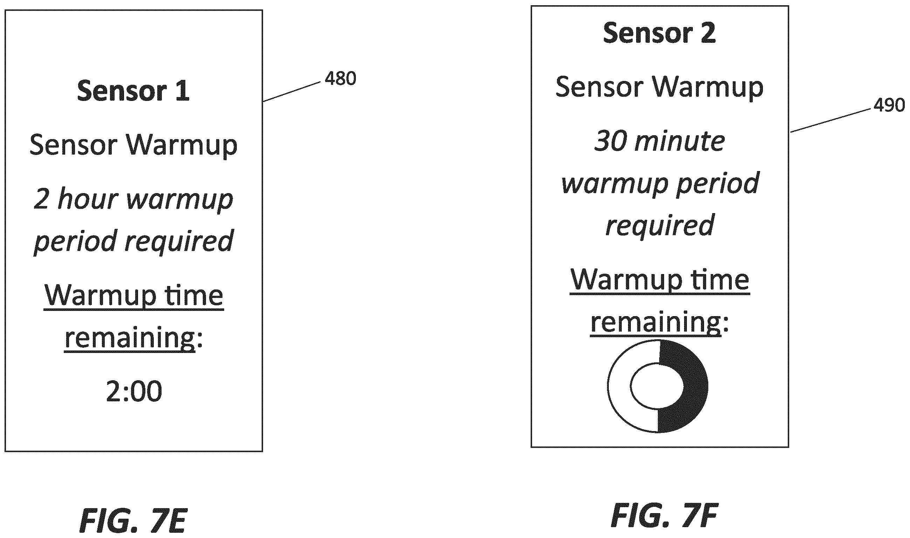

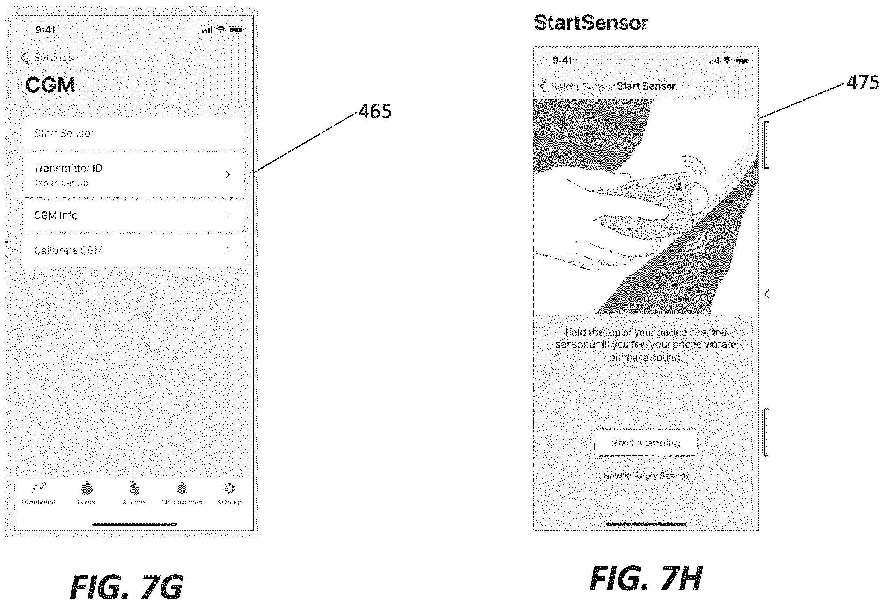

Upon selection of one of the sensor menu items 452, 454, 456, the user can be guided through configuring the sensor for use with the system as discussed herein. As noted above, different type of sensors may use different pairing, authentication and/or session initiation protocols. As such, in embodiments if the user selects Sensor 1 on the Select Sensor Screen 450 one or more menu screens guiding a user through the specific sequence required to activate, pair and initiate a CGM session for the type of sensor for Sensor 1 can be displayed and if the user selections Sensor 2 or Sensor 3 one or more menu screens that may be different from those for Sensor 1 and for each other may be displayed based on the specific requirements for those types of sensors. For example, the type of sensor of Sensor 1 may require an identifier such as a transmitter ID of the sensor to be entered to pair the sensor with the system and the type of sensor of Sensor 2 may detect such an identifier using Near Field Communication. As such, one of the menu screens displayed after selecting Sensor 1 can be menu screen such as screen 460 depicted in Figure 7C prompting the user to enter a Transmitter ID for the sensor into a dialogue box 462 as part of the process of pairing Sensor 1 with the system. Another screen 465 that can be displayed to guide a user through entering such an identifier is depicted in Figure 7G. Similarly, one of the menu screens after selecting Sensor 2 can be a menu screen such as screen 470 depicted in Figure 7D including an instruction to position the user's phone near to the sensor in order to detect a sensor identifier using NFC. Another screen 475 that can guide a user through scanning the sensor for an identifier is depicted in Figure 7H, which includes a graphical depiction of scanning the sensor to show the user the action that needs to be taken. As another example, as noted above different types of sensors can have different warmup periods. As such, different screens can be displayed depending on which sensor is selected informing the user of the warmup period and/or time remaining on the warmup period for the particular type of sensor with one or more of text, progress bar, status indicator, countdown, etc. as shown for example in menu screens 280 and 290 in Figures 7E and 7F.

Various other screens can be provided that are specific to the requirements of the type of sensor regarding sensor activation, authentication, session initialization, calibration etc.

In some embodiments, the system may automatically detect a type of sensor that the user will employ in the system. For example, the system could automatically detect the type of sensor when the user initiates a pairing procedure for pairing the sensor with the system.

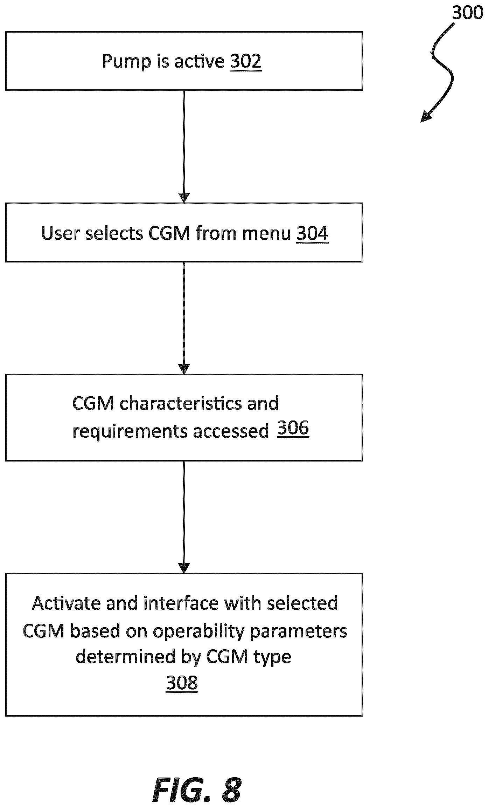

In FIG. 8, a method 300 demonstrates steps of configuring an infusion pump system to interface with a CGM sensor according to an embodiment. At step 302, a pump is activated and ready for use. At step 304, a user selects a CGM from a plurality of types of CGMs, each of which can be operably and communicatively coupled to the active pump. This may be done, for example, on menu screen 450 of Figure 7B. At step 306, the system can access the specific characteristics and requirements for the selected type of CGM. At step 308, the selected CGM will be activated and employed in the system based on the varying operability parameters, requirements, etc. of the selected type of CGM (e.g., activation protocol, communication protocol, type of data, authentication protocols, warmup period, data time interval, etc.).

In embodiments, an ambulatory infusion pump system can include a pump mechanism configured to deliver insulin to a user, a communications interface configured to receive data indicative of glucose levels of a user from one or more continuous glucose monitoring (CGM) sensors and a memory configured to store characteristics for each of a plurality of types of CGM sensors. At least one processor can be configured to receive an indication that a user will be using a type of sensor from the plurality of types of sensors. The at least one processor can the access the stored characteristics in memory for the type of sensor and configure the system to interface with the type of sensor based on the stored characteristics.

In some embodiments, the system further includes a user interface the indication that the user will using the type of sensor is received through the user interface.

In some embodiments, the at least one processor is further configured to display a menu on the user interface including the plurality of types of sensors and the indication is provided by the user selecting the type of sensor.

In some embodiments, the stored characteristics include an activation protocol required to activate each of the plurality of types of sensors for use with the system, and configuring the system to interface with the type of sensor based on the stored characteristics includes activating the type of sensor according to the corresponding activation protocol.

In some embodiments, the at least one processor is further configured to provide instructions to the user to guide the user through one or more steps of the activation protocol.

In some embodiments, the stored characteristics include a communication protocol employed by each of the plurality of types of sensors to transmit data to the communications interface, and configuring the system to interface with the type of sensor based on the stored characteristics includes configuring the communications interface to communicate with the type of sensor according to the corresponding communication protocol.

In some embodiments, the stored characteristics include a type of data transmitted by each of the plurality of types of sensors to the communications interface, and configuring the system to interface with the type of sensor based on the stored characteristics includes processing the data based on the type of data transmitted by the type of sensor.

In some embodiments, the stored characteristics include one or more authentication protocols required by each of the plurality of types of sensors, and configuring the system to interface with the type of sensor based on the stored characteristics includes implementing the one or more authentication protocols required by the type of sensor.

In some embodiments, the stored characteristics include a warmup period required by each of the plurality type of sensors following insertion before the type of sensor can reliably transmit data, and configuring the system to interface with the type of sensor includes coordinating communications with the type of sensor according to the warmup period.

In some embodiments, the stored characteristics include a time interval between communications of data indicative of a glucose level for each type of sensor, and configuring the system to interface with the type of sensor includes coordinating communications with the type of sensor based on the time interval.

In embodiments, an ambulatory infusion pump system can include a pump mechanism configured to deliver insulin to a user, a communications interface configured to receive data indicative of glucose levels of a user from one or more continuous glucose monitoring (CGM) sensors and a memory configured to store characteristics for each of a plurality of types of CGM sensors. A user interface can be configured to display a menu screen enabling a user to select a type of CGM sensor from a list of the plurality of types of CGM sensors and at least one processor can be configured to interface with the type of sensor based on the stored characteristics.

In some embodiments, the stored characteristics include an activation protocol required to activate each of the plurality of types of, and interfacing with the type of sensor based on the stored characteristics includes activating the type of sensor according to the corresponding activation protocol.

In some embodiments, the at least one processor is further configured to provide instructions to the user on the user interface to guide the user through one or more steps of the activation protocol.

In some embodiments, the stored characteristics include a communication protocol employed by each of the plurality of types of sensors to transmit data to the communications interface, and interfacing with the type of sensor based on the stored characteristics includes communicating with the type of sensor according to the corresponding communication protocol.

In some embodiments, the stored characteristics include a type of data transmitted by each of the plurality of types of sensors to the communications interface, and interfacing with the type of sensor based on the stored characteristics includes processing the data based on the type of data transmitted by the type of sensor.

In some embodiments, the stored characteristics include one or more authentication protocols required by each of the plurality of types of sensors, and interfacing with the type of sensor based on the stored characteristics includes implementing the one or more authentication protocols required by the type of sensor.

In some embodiments, the stored characteristics include a warmup period required by each of the plurality type of sensors following insertion before the type of sensor can reliably transmit data, and interfacing with the type of sensor includes coordinating communications with the type of sensor according to the warmup period.

In some embodiments, the stored characteristics include a time interval between communications of data indicative of a glucose level for each type of sensor, and interfacing with the type of sensor includes coordinating communications with the type of sensor based on the time interval.

In some embodiments, the user interface is part of an ambulatory infusion pump containing the pump mechanism.

In some embodiments, the user interface is part of a remote control device.

Various embodiments of systems, devices, and methods have been described herein. These embodiments are given only by way of example and are not intended to limit the scope of the claimed inventions. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the claimed inventions.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended.

Any incorporation by reference of documents above is limited such that no subject matter is incorporated that is contrary to the explicit disclosure herein. Any incorporation by reference of documents above is further limited such that no claims included in the documents are incorporated by reference herein. Any incorporation by reference of documents above is yet further limited such that any definitions provided in the documents are not incorporated by reference herein unless expressly included herein.

Also incorporated herein by reference in their entirety are commonly owned U.S. Patent Nos. 6,999,854; 8,133,197; 8,287,495; 8,408,421 8,448,824; 8,573,027; 8,650,937; 8,986,523; 9,173,998; 9,180,242; 9,180,243; 9,238,100; 9,242,043; 9,335,910; 9,381,271; 9,421,329; 9,486,171; 9,486,571; 9,492,608; 9,503,526; 9,555,186; 9,565,718; 9,603,995; 9,669,160; 9,715,327; 9,737,656; 9,750,871; 9,867,937; 9,867,953; 9,940,441; 9,993,595; 10,016,561; 10,201,656; 10,279,105; 10,279,106; 10,279,107; 10,357,603; 10,357,606; 10,492,141; 10/541,987; 10,569,016; 10,736,037; 10,888,655; 10,994,077; 11,116,901; 11,224,693; 11,291,763; 11,305,057; 11,458,246; 11,464,908; and 11,654,236 and commonly owned U.S. Patent Publication Nos. 2009/0287180; 2012/0123230; 2013/0053816; 2014/0276423; 2014/0276569; 2014/0276570; 2018/0071454; 2019/0307952; 2020/0206420; 2020/0329433; 2020/0368430; 2020/0372995; 2021/0001044; 2021/0113766; 2021/0353857; 2022/0062553; 2022/0139522; 2022/0223250; 2022/0233772; 2022/0233773; 2022/0238201; 2022/0265927; 2023/0034408; 2022/0344017; 2022/0370708; ; 2022/0037465; 2023/0040677; 2023/0047034; 2023/0113545 and 2023/0113755 and commonly owned U.S. Patent Applications Nos. 17/368,968; 17/896,492; 18/011,060; 18/071,814; 18/071,835; 18/075,029; 18/090,788 18/115,316; and 18/139,391.

For purposes of interpreting the claims, it is expressly intended that the provisions of 35 U.S.C. § 112(f) are not to be invoked unless the specific terms "means for" or "step for" are recited in a claim.

## Claims

1. An ambulatory infusion pump system, comprising:
a pump mechanism configured to deliver insulin to a user;
a communications interface configured to receive data indicative of glucose levels of a user from one or more continuous glucose monitoring (CGM) sensors;
a memory configured to store characteristics for each of a plurality of types of CGM sensors;
at least one processor configured to:
receive an indication that a user will be using a type of sensor from the plurality of types of sensors;
access the stored characteristics in memory for the type of sensor; and
configure the system to interface with the type of sensor based on the stored characteristics.

2. The ambulatory infusion pump system of claim 1, further comprising a user interface, and wherein the indication that the user will using the type of sensor is received through the user interface.

3. The ambulatory infusion pump system of claim 2, wherein the at least one processor is further configured to display a menu on the user interface including the plurality of types of sensors and the indication is provided by the user selecting the type of sensor.

4. The ambulatory infusion pump system of any preceding claim, wherein the stored characteristics include an activation protocol required to activate each of the plurality of types of sensors for use with the system, and wherein configuring the system to interface with the type of sensor based on the stored characteristics includes activating the type of sensor according to the corresponding activation protocol.

5. The ambulatory infusion pump system of claim 4, wherein the at least one processor is further configured to provide instructions to the user to guide the user through one or more steps of the activation protocol.

6. The ambulatory infusion pump system of any preceding claim, wherein the stored characteristics include a communication protocol employed by each of the plurality of types of sensors to transmit data to the communications interface, and wherein configuring the system to interface with the type of sensor based on the stored characteristics includes configuring the communications interface to communicate with the type of sensor according to the corresponding communication protocol.

7. The ambulatory infusion pump system of any preceding claim, wherein the stored characteristics include a type of data transmitted by each of the plurality of types of sensors to the communications interface, and wherein configuring the system to interface with the type of sensor based on the stored characteristics includes processing the data based on the type of data transmitted by the type of sensor.

8. The ambulatory infusion pump system of any preceding claim, wherein the stored characteristics include one or more authentication protocols required by each of the plurality of types of sensors, and wherein configuring the system to interface with the type of sensor based on the stored characteristics includes implementing the one or more authentication protocols required by the type of sensor.

9. The ambulatory infusion pump system of any preceding claim, wherein the stored characteristics include a warmup period required by each of the plurality type of sensors following insertion before the type of sensor can reliably transmit data, and wherein configuring the system to interface with the type of sensor includes coordinating communications with the type of sensor according to the warmup period.

10. The ambulatory infusion pump system of any preceding, wherein the stored characteristics include a time interval between communications of data indicative of a glucose level for each type of sensor, and wherein configuring the system to interface with the type of sensor includes coordinating communications with the type of sensor based on the time interval.

11. The ambulatory infusion pump system of any of claims 1-10, wherein the user interface is part of an ambulatory infusion pump containing the pump mechanism.

12. The ambulatory infusion pump system of any of claims 1-10, wherein the user interface is part of a remote control device
